Europäisches Patentamt

European Patent Office

Office européen des brevets

(19)

(11) Veröffentlichungsnummer: **0 284 802**
A2

(12) **EUROPÄISCHE PATENTANMELDUNG**

(21) Anmeldenummer: 88103152.0

(22) Anmeldetag: 02.03.88

(51) Int. Cl.⁴: **A61B 19/00** , A61F 7/00 , A61K 9/50 , A61K 47/00

(30) Priorität: 28.03.87 DE 3710371

(43) Veröffentlichungstag der Anmeldung:
05.10.88 Patentblatt 88/40

(84) Benannte Vertragsstaaten:
CH DE ES FR GB IT LI SE

(71) Anmelder: DORNIER MEDIZINTECHNIK GMBH
Postfach 1128
D-8034 Germering 1(DE)

(72) Erfinder: Wilke, Joachim, Dr.
Angerhofstrasse 14
D-8034 Germering(DE)

(74) Vertreter: Landsmann, Ralf, Dipl.-Ing.
DORNIER GMBH - Patentabteilung - Kleeweg 3
D-7990 Friedrichshafen 1(DE)

(54) Zerstörung von krankhaft veränderten Gewebezellen.

(57) Die Erfindung betrifft die Zerstörung pathogener Gewebswucherungen, wie beispielsweise Sakome oder Melanome, wobei die krankhaften Zellen mit kolloidalen Metallpartikel markiert werden und anschliessend einer extrakorporalen Applikation fokussierter Stosswellen oder einer Ultraschallapplikation ausgesetzt werden.

Fig. 2

EP 0 284 802 A2

## Zerstörung von krankhaft veränderten Gewebezellen

Die Erfindung betrifft die Zerstörung markierter, krankhafter Zellen unter Ausnutzung der Stosswellen-und/oder Ultraschalltechnologie.

Aus der DE-PS 23 51 247 ist bekannt, Stosswellen zur Zertrümmerung von in Körpern von Lebewesen befindlichen Konkrementen zu verwenden, wobei eine Unterwasserfunkenstrecke in einem Brennpunkt eines Rotationsellipsoiden eine Stosswelle erzeugt, die im anderen Brennpunkt fokussiert wird. Eine Fokussierkammer als Teil eines Rotationsellipsoiden ist dabei mit einer Flüssigkeit gefüllt und mittels der Funkenstrecke wird durch elektrische Unterwasserfunkenentladung in einem Kondensator gespeicherte Energie in mechanische Stosswellenenergie umgewandelt.

Die Erzeugung von Stosswellen ist auch auf elektroinduktivem Wege möglich.

In der DE-OS 33 12 014 ist ein Stosswellenrohr beschrieben, bei dem eine kugelkalottenförmige Spule vorgesehen ist, an der eine Membran anliegt, über die die Stosswellen auf ein Übertragungsmedium abgegeben werden. Die Fokussierung der Stosswellen geschieht hier durch die kegelstumpfartige Formgebung der Vorrichtung. Darüber hinaus können Stosswellen piezoelektrisch oder mittels Laserstrahlung induziert werden.

Aus dem Bereich der Elektronenmikroskopie ist ein Verfahren bekannt, durch das Zellen mit kolloidalem Gold, das mit Proteinen (z.B.: Antikörpern, Lektinen etc.) beladen ist, die für membranständige Biomoleküle spezifisch sind, markiert werden (Horisberger und Rosset in "The Journal of Histochemistry and Cytochemistry", Seite 295 - 305, 1977). Hierbei werden überwiegend Goldkugeln mit einem Durchmesser von 5 bis 25 nm verwendet, aber auch Goldkolloide mit einem Durchmesser von ca. 150 nm wurden beschrieben (Frens in "Nature Physical Science, Vol. 241, Seite 20 - 22", 1973). Ein Verfahren zur Herstellung von Gold-Liposomen ist ebenfalls bekannt (Hong et al., in "Biochimica et Biophysica Acta", Seite 320 - 323, 1983).

Eine Zerstörung von Tumorzellen in vitro unter Verwendung von kolloidalen Metallpartikeln und Ultraschall wurde von Wagai und Kikuchi (in "Ultrasonic Energy", Seite 179 - 189, 1965) beschrieben.

Der Erfindung liegt die Aufgabe zugrunde, krankhafte Zellen in Körpern von Lebewesen zu zerstören ohne dabei umliegendes, gesundes Gewebe zu gefährden und in vitro beispielsweise kanzerogene Blutzellen selektiv zu eliminieren.

Die Aufgabe wird durch die Erfindung nach Anspruch 1 und den weiteren Ansprüchen gelöst.

Die weltweit zur Nierensteinzertrümmerung angewandte Stosswellentechnologie wird erfindungsgemäss zur Zerstörung krankhafter Gewebezellen angewandt. Die krankhaften Körperzellen werden mit kolloidalen Edelmetallpartikeln, vorzugs weise Goldpartikeln, markiert, wobei die Partikel an der Zellmembran anhaften. Die Verwendung von Edelmetallen verhindert das Auftreten von unerwünschten Nebenreaktionen. Die Metallpartikel können auch in Lipidvesikeln oder Liposomen eingeschlossen werden, die sich an den wuchernden Stellen anlagern.

Die Durchmesser der Lipidvesikeln variieren je nach Lipid, Lipidgemisch und Herstellungsweise zwischen 200 Å und 100 µm. Spritzt man diese Lipidvesikel bzw. Liposomen intravenös, verteilen sich diese über den ganzen Körper.

Durch fokussierte Beschallung eines Zielvolumens, welche die Lipidvesikel oder Liposomen vorübergehend öffnet, kann das eingeschlossene Metallpartikel in Abhängigkeit von der Anzahl der Stosswellen dosiert und örtlich definiert freigesetzt werden. Bei ausreichend durchbluteten Organen (z.B. Niere, Leber) werden ständig neue Lipidvesikel oder Liposomen, die partikelbeladen sind, über dem Blutkreislauf nachgeliefert.

Depoteffekte der Lipidvesikel oder Liposomen und damit der eingeschlossenen Partikel können mit mehreren Methoden erreicht werden.

Die Lipidmembran der Vesikel oder Liposomen kann durch oberflächenaktive Substanzen derart manipuliert werden, dass diese sich gezielt an Strukturen im Zielorgan anlagern. Durch das Öffnen der Vesikel oder Liposomen mittels Stosswellen kann lokal eine Metallpartikel-Freisetzung erreicht werden.

Die Grösse der Lipidvesikel oder Liposomen in Kombination mit einem gezielten Applikationsweg, kann bedingt durch Filtration (Passage zu grosser Vesikel/Liposomen nicht möglich) im Blutkreislauf zu lokalen Anreicherungen führen. Das Öffnen dieser angereicherten Lipidvesikel/Liposomen erfolgt mittels Stosswellen. Für eine Anzahl medizinischer Anwendungen kann eine fraktionierte Freisetzung von Vorteil sein. Dies ist durch Beschallen des Zielvolumens in nahezu beliebigen Zeitintervallen möglich.

Statt der Liposomen können auch metallpartikelhaltige Kunststoffsphären mit einem Durchmesser im Nano-bis Mikrometerbereich Verwendung finden.

Die Stosswelle wird in bekannter Weise auf die edelmetallmarkierten, krankhaften Zellregionen fokussiert. Aufgrund diffuser Reflexionen an den Metallpartikeln infolge ihrer relativ zum Körpergewebe andersartigen physikalischen Eigenschaften tritt eine Rayleigh-Streuung der Stosswellen auf. Wesentlich für die Schädigungswirkung ist die hierbei auftretende Relativgeschwindigkeit zwischen den Streuzentren und dem Umgebungsmedium. Infolge der gegenläufigen Bewegung resultiert eine lokale Zerstörung der Zellmembran, deren Ausmaß von der applizierten Intensität, von der Grösse der Edelmetallkugeln und von der Anzahl der Kugeln bzw. ihrer Verteilung auf der Zellmembran abhängig ist. Ein Eindringen der Kugeln in die Zelle wirkt sich negativ auf die osmotische Stabilität und die innere Struktur der Zelle aus. Das Ausströmen von Ionen oder intrazellulärer Proteine ist ebenso möglich wie die Zerstörung von für die Zelle lebenswichtigen Organellen, z.B. des Zellkerns.

Neben einer Behandlung mit fokussierten Stosswellen ist auch eine Anwendung von Ultraschallbehandlungsmethoden oder eine Kombination von Stosswellen-und Ultraschalltherapie möglich.

Besonders geeignet ist das Verfahren zur Behandlung von erkranktem Endothelgewebe, das von den applizierten Metallkolloiden gut erreicht werden kann. Auch die Therapie von Lebertumoren oder anderer solider und schwer zu operierender Tumore ist möglich. Für die Behandlung ist es sinnvoll, die proteinbeladenen Edelmetallpartikel oder mit Metallpartikeln beladenen Lipidvesikeln oder Kunststoffsphären möglichst nahe dem zu zerstörenden Gewebe zu injizieren.

Ergänzend oder alternativ zum beschriebenen Verfahren ist es möglich, die Edelmetallpartikel mit Protein-Toxin Konjugaten zu beladen. Die Metallpartikel können somit als Träger dienen, um hochwirksame Zellgifte, für die die Membran aber nicht permeabel ist, in die Zelle zu transportieren.

Ein Vorteil der Tumortherapie mit biochemisch beschichteten kolloidalen Edelmetallen und Stoss- oder Ultraschallwellen ist, dass nicht notwendigerweise ein tumorspezifischer Market bekannt sein muß. Die zur Beschichtung der Edelmetallkugeln verwendeten Biomoleküle können auch gegen Membranstrukturen, die für das Gewebe spezifisch sind dem der Tumor entstammt, gerichtet sein. Die spezifische Zerstörung der Tumorzellen wird durch die Fokussierung der Stosswellen bzw. durch eine gezielte Ultraschallapplikation gewährleistet. Die Zerstörung pathogener Zellen kann sowohl in vivo als auch in vitro vorgenommen werden.

In vitro erlaubt die Verwendung magnetischer Metalle zusätzlich eine partielle Präzipitation der zerstörten Zellen mittels Magneten.

In der klinischen Praxis lässt sich oben beschriebene Vorgehensweise beispielsweise zur Eliminierung kanzerogener Blutzellen oder bei Knochenmarkstransplantationen zur Entfernung immunkompetenter T-Lymphozyten aus dem Knochenmarksblut des Spenders verwenden. Die zuverlässige Elimination dieser Zellen ist eine Voraussetzung, um Knochenmarkstransplantationen von einem Elternteil zum Kind zu ermöglichen. Darüberhinaus ist das Verfahren in der experimentellen Forschung zur negativen Selektion von Zellen nützlich.

Die Erfindung wird nachfolgend anhand von Figuren näher beschrieben.

Es zeigen:

Figur 1 eine Lipiddoppelschicht,

Figur 2 eine aus einer Doppellipidschicht gebildete Vesikel,

Figur 3 eine Einrichtung zur Freisetzung von Metallpartikeln mit Stosswellen,

Figur 4 eine weitere Einrichtung in anderer Bauweise und

Figur 5 eine Einrichtung zur Freisetzung von Metallpartikeln mit Ultraschall und Stosswellen.

Der Aufbau biologischer Membranen hat in dem Modell der "fluid-mosaic-membrane" seine heute allgemein akzeptierte Form gefunden.

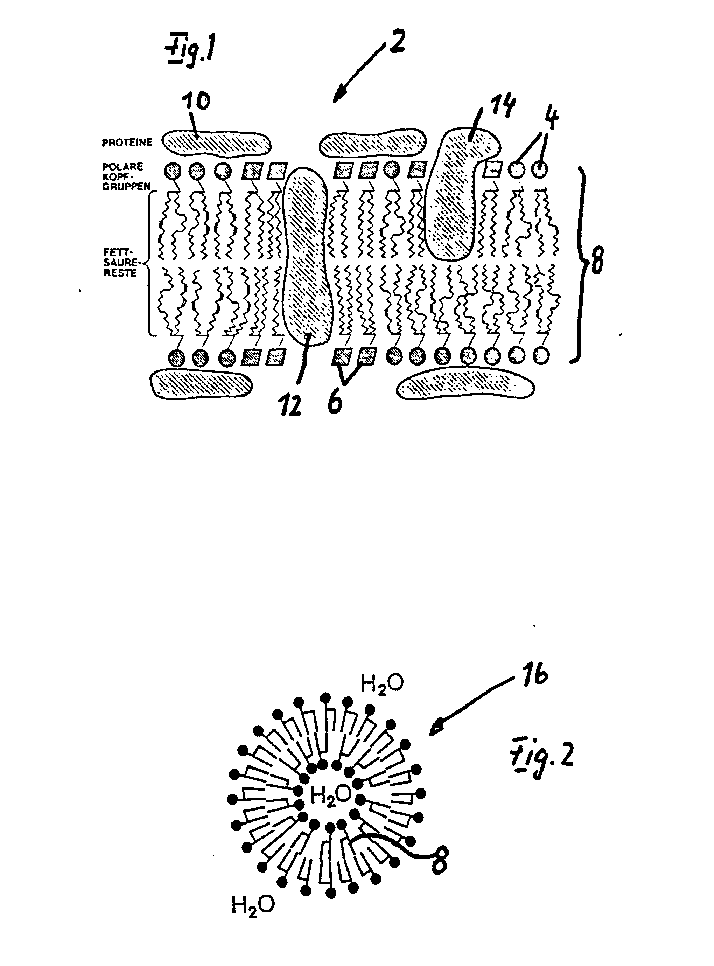

Lipidmoleküle 2 mit hydrophilen Enden 4 (polar) und hydrophoben Enden 6 (unpolar) formen eine Lipiddoppelschicht 8 der Art, dass die hydrophoben Enden einander zugewandt und die hydrophilen Köpfe die Grenzfläche zur jeweils wässrigen Phase bilden. Die beiden Schichten sind dementsprechend antiparallel orientiert, wie Figur 1 zeigt. Des weiteren ist es möglich, Proteine 10, 12 und 14 in die Vesikelmembran einzubauen oder anzulagern.

Beim Aufbau von Vesikeln unterscheidet man zwischen einschaligen (aus einer Doppellipidschicht) und mehrschaligen (aus mehreren Doppellipidschichten bestehend) Vesikeln.

Figur 2 zeigt das Modell einer Vesikel 16, wobei die Vesikel aus einer Doppellipidschicht 8 gebildet ist.

Die Grösse einer einschaligen Vesikel variiert zwischen 200 und 1000 Å in Abhängigkeit von der Herstellungsweise und den benutzten Lipiden. Eine Erhöhung der Membrandicke (lange Fettsäureketten) führt zu grösseren Vesikeldurchmessern. Mehrschalige Vesikel können bis zu einer Grösse von max. 100 μm hergestellt werden.

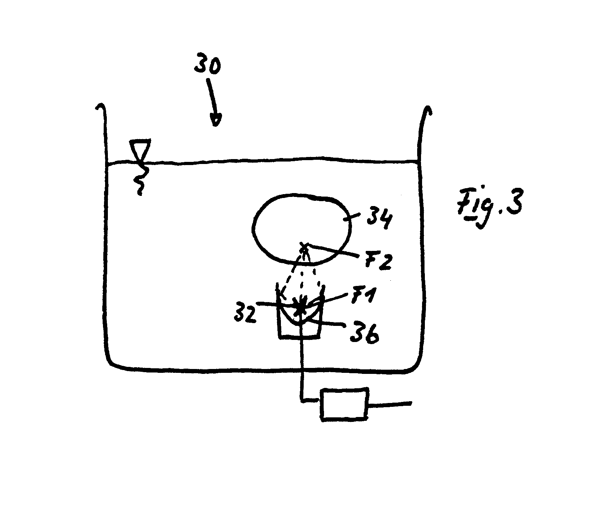

Figur 3 zeigt eine Einrichtung zur Freisetzung von Metallpartikeln in prinzipieller Darstellung. Innerhalb einer wassergefüllten Wanne 30 befinden sich eine Stosswellenquelle 32 und ein Patient 34. Die Stosswellenquelle befindet sich in Ausführungsbeispiel im Brennpunkt F1 eines Rotationsellipsoiden 36. Von der Stosswellenquelle ausgehende Stosswellen werden im Fokus F2 konzentriert. Dort befindet sich das krankhafte Gewebe und die Lipidvesikeln, aus denen Metallpartikel freigesetzt werden sollen.

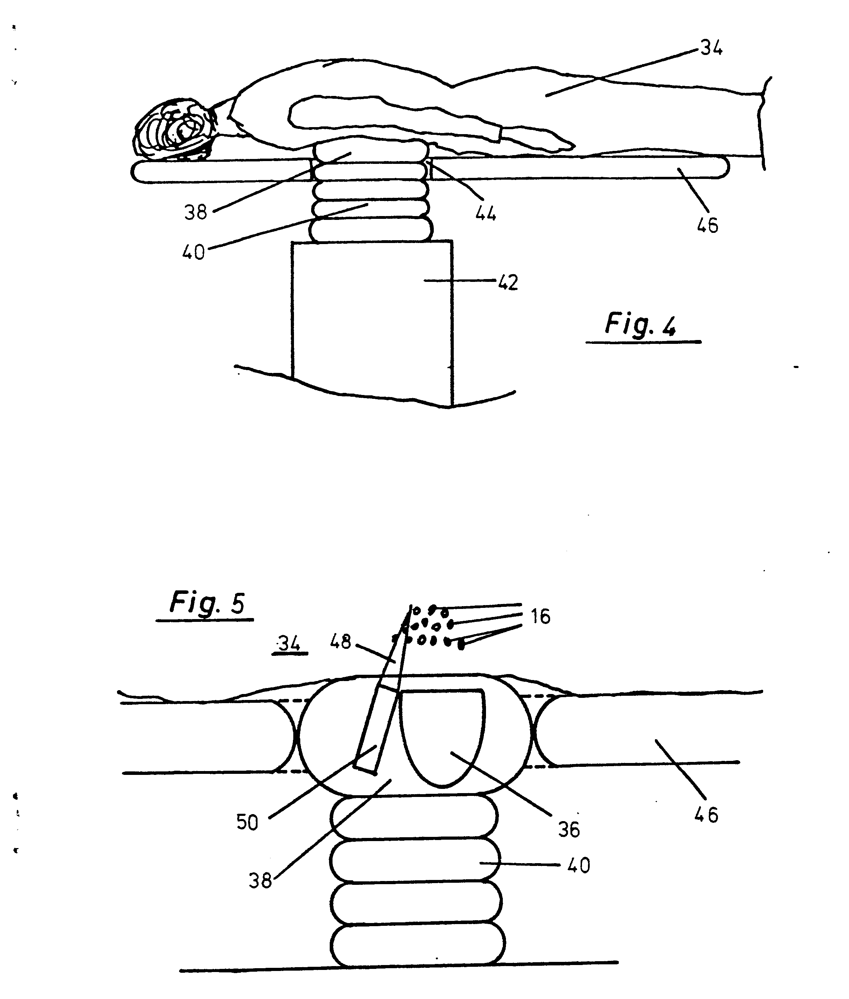

Figur 4 zeigt eine Einrichtung, bei der der Patient 34, hier in Bauchlage dargestellt, auf einem Wasserkissen 38 aufliegt. Das Wasserkissen 38 ist durch einen Faltenbalg 40 mit einem Stosswellenerzeuger 42 verbunden. Durch eine Aussparung 44 in einer Patientenliege 46 hindurch gelangt das Wasserkissen 38 bis an den Patienten 34 heran.

Figur 5 zeigt die Anwendungsmöglichkeit, dass eine Stosswelleneinrichtung und eine Ultraschalleinrichtung innerhalb des Wasserkissen 38 angeordnet sind. Dabei kann der Schallkegel 48 eines Ultraschallschwingers 50 auf den Punkt ausgerichtet sein, der den zweiten Brennpunkt eines Rotationsellipsoiden 36 bildet und indem sich das krankhafte Gewebe und die Liposomen 16 befinden.

Zur Erzeugung von Stosswellen können Funkenentladung, Laser, elektromechanische Quellen oder piezoelektrische Erzeuger verwendet werden. Zur Fokussierung können an Stelle eines Ellipsoiden auch Kugelkalotten oder Linsenfokussierung dienen.

Die Behandlung von Tumoren mit kolloidalen Metallpartikeln und Stosswellen oder Ultraschall kann ohne weiteres, insbesondere zur Bekämpfung von Tumorresten, durch eine Röntgen-, Chemo- und/oder Hyperthermiebehandlung und/oder eine Injektion von Gasen oder Partikeln ergänzt werden. Gleichzeitig können neben den metallpartikelbeladenen Liposomen zur Unterstützung der Behandlung auch pharmakabeladene Liposomen verwendet werden.

## Ansprüche

1. Zerstörung von biologischen Zellen, **dadurch gekennzeichnet**, dass die Zellen mit Metallpartikeln markiert und einer Stosswellen- und/oder Ultraschallapplikation ausgesetzt werden.

2. Zerstörung von Zellen in vivo nach Anspruch 1, dadurch gekennzeichnet, dass die Applikationen extrakorporal erzeugt werden.

3. Zerstörung von Zellen nach Anspruch 1, dadurch gekennzeichnet, dass die Stosswellen mittels eines fokussierten Laserstrahls nahe den mit kolloidalen Metallpartikeln markierten Zellen mit Hilfe eines Lichtleiters erzeugt werden.

4. Zerstörung von Zellen nach Anspruch 1, dadurch gekennzeichnet, dass die markierten Zellen einem stationären Ultraschallfeld in Verbindung mit gleichzeitiger Stosswellenapplikation ausgesetzt werden.

5. Zerstörung von Zellen nach wenigstens einem der Ansprüche 1 bis 4, dadurch gekennzeichnet, dass für die Markierung der Zellen die Verwendung von in Liposomen eingeschlossenen Metallpartikeln vorgesehen ist.

6. Zerstörung von Zellen nach wenigstens einem der Ansprüche 1 bis 4, dadurch gekennzeichnet, dass für die Markierung der Zellen die Verwendung von metallpartikelhaltigen Kunststoffpartikeln vorgesehen ist.

7. Zerstörung von Zellen nach wenigstens einem der Ansprüche 1 bis 6, dadurch gekennzeichnet, dass für die Markierung der Zellen die Verwendung biochemisch beschichteter Metallpartikel vorgesehen ist.

8. Zerstörung von Zellen nach wenigstens einem der Ansprüche 1 bis 7, dadurch gekennzeichnet, dass die Metallpartikel Träger intrazellulär wirksamer Toxine sind.

9. Zerstörung von Zellen nach wenigstens einem der vorherigen Ansprüche, dadurch gekennzeichnet, dass für die Beschichtung der Metallpartikel Biomoleküle bzw. deren Hapten-oder Toxin-Konjugate vorgesehen sind.

10. Zerstörung von Zellen in vivo nach wenigstens einem der vorherigen Ansprüche, dadurch gekennzeichnet, dass die Metallpartikel, die metallpartikelbeladenen Liposomen oder die metallpartikelhaltigen Kunststoffpartikel möglichst nahe dem zu zerstörenden Gewebe injiziert werden.

11. Zerstörung von Zellen nach wenigstens einem der vorherigen Ansprüche, dadurch gekennzeichnet, dass die an der Zellmembran anhaftenden Metallpartikel, metallpartikelbeladenen Liposomen oder metallpartikelbeladenen Kunststoffpartikel in das Zellinnere getrieben werden.

12. Zerstörung von Zellen in vitro nach wenigstens einem der vorherigen Ansprüche, dadurch gekennzeichnet, dass die zur Markierung verwendeten Metallpartikel magnetisch sind.

13. Zerstörung von Zellen nach wenigstens einem der vorherigen Ansprüche, dadurch gekennzeichnet, dass die Metallpartikel aus Edelmetall, z.B. Gold, Silber oder Platin bestehen.

14. Zerstörung von Zellen nach Anspruch 13, dadurch gekennzeichnet, dass die Metallpartikel vorzugsweise aus Gold bestehen.

15. Eliminierung von Zellen in vitro, dadurch gekennzeichnet, dass die mit magnetischen Metallpartikeln, metallpartikel beladenden Liposomen oder metallpartikelhaltigen Kunststoffpartikeln beladene und durch die Stosswellen-und/oder Ultraschallbehandlung geschädigte Zellen mittels Magnet präzipitiert werden.

16. Zerstörung von pathogenen Gewebswucherungen nach einem der vorherigen Ansprüche, dadurch gekennzeichnet, dass die Stosswellen-und/oder Ultraschallwellenapplikationen in Kombination mit einer Röntgen-, Chemo-und/oder Hyperthermiebehandlung anwendbar sind.

17. Freisetzung von in Lipidvesikeln enthaltenen Metallpartikeln, dadurch gekennzeichnet, dass die gewünschte Menge der freizusetzenden Partikel mittels einer einmaligen Stosswellenbehandlung und/oder Ultraschallbehandlung begrenzter Dauer freigesetzt wird.

18. Freisetzung von in Lipidvesikeln enthaltenen Metallpartikeln nach Anspruch 17, dadurch gekennzeichnet, dass die gewünschte Menge der freizusetzenden Partikel mittels wiederholter oder dauernder Stosswellen-und/oder Ultraschallbehandlung freigesetzt wird.

19. Freisetzung von in Lipidvesikeln enthaltenen Metallpartikeln nach Ansprüchen 17 und 18, dadurch gekennzeichnet, dass die Stosswellenbehandlung und/oder Ultraschallbehandlung immer dann fortgesetzt wird, wenn sich frisches Blut mit Metallpartikeln enthaltenen Lipidvesikeln im Gewebe befindet.

20. Freisetzung von in Lipidvesikeln enthaltenen Metallpartikeln nach wenigstens einem der vorherigen Ansprüche, dadurch gekennzeichnet, dass die Intensität der Stosswellenbehandlung und/oder Ultraschallbehandlung variierbar ist, wobei der Druckgradient geringer ist als die Zerstörungsschwelle lebenden Gewebes.

21. Freisetzung von in Lipidvesikeln enthaltenen Metallpartikeln nach wenigstens einem der vorherigen Ansprüche, dadurch gekennzeichnet, dass mindestens eine fokussierte Stosswellenquelle oder Ultraschallquelle vorhanden ist und der Brennfleck variabel gewählt werden kann.

22. Freisetzung von in Lipidvesikeln enthaltenen Metallpartikeln nach wenigstens einem der vorherigen Ansprüche, dadurch gekennzeichnet, dass mehrere fokussierte Stosswellenquellen und/oder Ultraschallquellen vorhanden sind und die Wellenfront variabel gestaltet werden kann.

23. Lipidvesikel oder Lipidgemische für die berührungsfreie Freisetzung von Metallpartikeln mittels Stosswellen und/oder Ultraschall, gekennzeichnet durch Lipidmoleküle mit hydrophilen und hydrophoben Enden, die derart eine Lipiddoppelschicht bilden, dass die hydrophoben Enden einander zugewandt und die hydrophilen Köpfe die Grenzfläche zur jeweils wässrigen Phase der Umgebung bilden und sich im Membraninneren Metallpartikel befinden.

24. Lipidvesikel oder Lipidgemische für die berührungsfreie Freisetzung von Metallpartikeln mittels Stosswellen, bestehend aus Lipidmolekülen mit hydrophilen und hydrophoben Enden, die derart eine Lipiddoppelschicht bilden, dass die hydrophoben Enden einander zugewandt und die hydrophilen Köpfe die Grenzfläche zur jeweils wässrigen Phase der Umgebung bilden und sich im Membraninneren Metallpartikel befinden, **dadurch gekennzeichnet**, dass Fehlstellen in die Vesikelmembran eingebaut sind, die die Freisetzung der Partikel bei Stosswellen-und/oder Ultraschallbeaufschlagung begünstigen oder erleichtern.

25. Lipidvesikel für die berührungsfreie Freisetzung von Metallpartikeln mittels Stosswellen und/oder Ultraschall nach Anspruch 24, dadurch gekennzeichnet, dass die Fehlstellen an die Vesikelmembran angelagert sind.

26. Lipidvesikel für die berührungsfreie Freisetzung von Metallpartikeln mittels Stosswellen und/oder Ultraschall nach Ansprüchen 24 und 25, dadurch gekennzeichnet, dass die Fehlstellen Proteine sind.

27. Lipidvesikel für die berührungsfreie Freisetzung von Metallpartikeln mittels Stosswellen und/oder Ultraschall nach wenigstens einem der vorherigen Ansprüche, dadurch gekennzeichnet, dass die Lipidmembran oberflächenaktive Substanzen aufweist, die derart manipuliert sind, dass sich die Lipidvesikel in einem bestimmten Organ oder Organteil anlagern.

28. Lipidvesikel für die berührungsfreie Freisetzung von Metallpartikeln mittels Stosswellen nach Anspruch 27, dadurch gekennzeichnet, dass sich die Lipidvesikel aufgrund ihrer Grösse und/oder ihres Applikationswegs in einem Organ oder Organteil anreichern.

29. Unterstützung der berührungsfreien Freisetzung von Metallpartikeln, die sich in Lipidvesikeln im lebenden Gewebe befinden, gekennzeichnet durch lokale Hyperthermie, Röntgen-und/oder Chemoexposition oder Injektion von Gasen oder Partikeln, die eine Vielfachreflexion der Stoss-und/oder Ultraschallwellen im Zielvolumen bewirken.

30. Vorrichtung zur Freisetzung von Metallpartikeln, die sich in Lipidvesikeln im lebenden Gewebe befinden, gekennzeichnet durch eine Einrichtung, die fokussierte Stosswellen aussendet und zu dem zu behandelnden Gewebe leitet.

31. Vorrichtung nach Anspruch 30, dadurch gekennzeichnet, dass die Stosswellen elektromagnetisch, piezoelektrisch, mit Laser oder einer Funkenstrecke erzeugt werden und mittels einer Linse, eines Linsensystems, einer Fokussierkammer oder Kugelkalotten fokussiert werden.

32. Vorrichtung nach wenigstens einem der vorherigen Ansprüche, gekennzeichnet durch einen elastischen Faltenbalg mit Wasserkissen für die Einkopplung der Stosswelle.

33. Vorrichtung zur Freisetzung von Metallpartikeln, die sich in Lipidvesikeln im lebenden Gewebe befinden können, dadurch gekennzeichnet, dass ein Ultraschallschwinger, beispielsweise an einem freibeweglichen Arm befestigt, zur Aussendung von Ultraschallwellen vorgesehen ist.

Fig. 1

Fig. 2

Fig. 3

Fig. 4

Fig. 5